# EUROPEAN PATENT APPLICATION

(11) **EP 3 494 796 A1**
(43) Date of publication of application: **12.06.2019**
(21) Application number: 17205344.9
(22) Date of filing: 05.12.2017
(51) Int. Cl.: A23D 7/005, A23D 7/00, A23L 35/00, A23L 33/115, A23L 33/105, A61K 8/92, A23D 7/06, C11B 1/00, C11B 1/10

(54) **OXIDATIVE STABILITY OF OLEOSOMES**

(71) Applicant: Cargill, Incorporated, Wayzata, MN 55391 (US)
(72) Inventor: The designation of the inventor has not yet been filed
(74) Representative: Elseviers, Myriam

(57) **Abstract**

The present invention relates to extracted or isolated high oleic seed oleosomes with an increased oxidative stability, a process for preparing it and the use in finished products.

## Description

### Field of the Invention

The present invention relates to oxidative stable high oleic seed oleosomes, the process for preparing them and the use in finished products.

### Background of the Invention

Emulsions, which are mixtures of two mutually insoluble components, the most known being oil and water, are widely used in formulations of various products. Commonly known domestic examples of emulsion-based formulations include mayonnaise, spreads, creamers, beverages, margarine and frozen desserts.

Although emulsions are providing to products containing them many advantageous properties, the present inventors discovered that their benefits are not fully leveraged.

There is still a need for emulsions which have good or improved oxidative stability. The current invention is fulfilling the need.

### Summary of the Invention

The current invention relates to extracted or isolated and oxidative stable high oleic seed oleosomes characterized in that the oleosomes are comprising high oleic seed oil with a fatty acid profile comprising at least 65% oleic acid, and the oxidative stability of the oleosomes is increased with at least 50%, preferably at least 70%, more preferably at least 100% in comparison to the oxidative stability of the corresponding seed oil.

It relates to a food, feed or personal care product comprising the oleosomes according to the present invention.

It further relates to the use of the oleosomes of the present invention for its anti-oxidative properties in food, feed or personal care product.

Finally it relates to a process for preparing the oleosomes of the present invention and it is comprising the following steps:
a) Separating a suspension of seeds and water in a ratio of 1:0.5 up to 1:10, into a hydrophobic-like phase and a hydrophilic-like phase by applying a gravity-based sedimentation force, and
b) Washing at least two times with water in a ratio of 1:0.5 up to 1:10 hydrophobic-like phase to water for obtaining the oleosomes,
c) Optionally repeating the step b),
   and
   wherein the suspension has a pH of from 6.5-8, preferably from 7 - 7.8, more preferably from 7.3-7.7, from 7.4 to 7.5.

### Detailed Description

The current invention relates to extracted or isolated and oxidative stable high oleic seed oleosomes characterized in that the oleosomes are comprising high oleic seed oil with an fatty acid profile comprising at least 65% oleic acid, and the oxidative stability of the oleosomes is increased with at least 50%, preferably at least 70%, more preferably at least 100% in comparison to the oxidative stability of the corresponding seed oil.

The oleosomes of the present invention are isolated or extracted from the corresponding high oleic seeds and are also known as "oil bodies", "lipid bodies", "lipid droplets" or "spherosomes". They are pre-emulsified droplets or vesicles of oil stored in plant seeds and are used as energy source for plant growth and metabolism. The droplets are stabilised by a layer containing phospholipids and various proteins associated with the oleosomes generically called "intrinsic proteins", said intrinsic proteins containing mostly oleosins. Caleosin and stereolosin are minor intrinsic proteins. The oleosins contain a hydrophilic part, which is present at the oleosomes' surface and a hydrophobic part which is anchored in the oil and ensures for oleosomes stability. The oil contained by the oleosomes contains a mixture of triglycerides of which the exact composition depends on the plant species from which the oil is derived. Beyond these major components, phospholipids, sterols, sterol-esters, and/or tocopherols may be present as minor components. These minor compounds can be present in an amount of 0.1 to 4 wt%, preferably from 0.5 to 2 wt%.

The oleosomes of the present invention have an increase of the oxidative stability of at least 50%, preferably 70%, more preferably at least 100% compared with the oxidative stability of the corresponding seed oil. The measurement is not bound by a specific method, but the oleosomes of the current invention show the increase of oxidative stability when replacing the oleosomes of the present invention with the corresponding seed oil in a weight to weight ratio.
The oleosomes of the present invention have an oxidation induction period of at least 150 h, preferably at least 170 h, measured according to ASTM D525 - 05 performed at 70°C and at 5 bar oxygen pressure. Measured at a temperature of 110°C and at 5 bar oxygen pressure, the oxidation induction period is at least 8h, at least 9h, preferably at least 10h. In general, the equipment known as Oxipress can be used to perform the analytical method. The oxidation induction period describes the onset of the oxidation. By performing the measurement at elevated temperatures, e.g. 70°C or 110°C, the onset is accelerated and it is providing in a shorter period of time the assessment of the oxidation stability. The person skilled in the art can extrapolate the oxidation induction period measured at elevated temperatures to the effective oxidation stability at ambient temperature.

In general the dry matter content of the oleosomes is from 50 to 100%, at least 60% up to 99%, at least 70% up to 80%.

The high oleic seed can be obtained from different seed varieties either obtained by natural selection or by genetic modifications (GMO).
In particular the high oleic seed varieties are obtained from seeds of sunflower, rapeseed, canola, camelina, olive, safflower, soybean, corn germ, preferably sunflower. These high oleic varieties are containing at least 65%, at least 70%, at least 75%, preferably at least 80% oleic acid in respect of the fatty acid profile. More specifically the high oleic variety of sunflower is containing at least 65%, at least 70%, at least 75%, preferably at least 80% oleic acid in respect of the fatty acid profile.

Without being bound to any theoretical explanation, the inventors have surprisingly found that the increased oxidative stability of the oleosomes of the present invention can be particularly useful when beyond the oleosomes of the present invention, additional proteins are present, more in particular proteins that accelerate the onset of oxidation. Examples of such additional proteins may be iron containing egg yolk and/or lactoferrin and/or transferrin both present in milk.

Surprisingly it has been found that the presence of egg yolk in the oleosomes of the present invention, does not accelerate the onset of oxidation.

Furthermore, it has been found that by using the oleosomes of the present invention, in presence of lactoferrin and/or transferrin present in milk, the onset of oxidation is not accelerated and furthermore, no extra homogenisation step is needed.

The present invention further relates to a food, feed or personal care product comprising the oleosomes of the present invention.
These products may be presented in a wide array of forms, including but not limited to a powder, a cream, a gel, a waxy solid, a paste, a liquid and the like.
In one aspect of the invention, products in the form of a liquid or in a low viscous form benefit from the oxidative stability of the oleosomes of the present invention.

The products, such as food, feed or personal care product of the present invention are characterized in that they contain an analytical detectable amount of oleosin, due to the presence of oleosomes. Any method suitable to analyze small amounts of proteins is suitable to detect the presence of oleosin in these products.

The personal care products of the present invention are formulations for topical application such as skin care products, cosmetic products, exfoliating products, topically applied veterinary products, and the like. These products benefit from the oxidative stability of the oleosomes of the present invention.

The feed product is encompassing in particular aquafeed and pet food. The use of the oleosomes of the present invention in these feed products may reduce the use of anti-oxidants such as ethoxyquin that is used particularly in pet food to reduce rancidification of fats.

The food product of the present invention is selected from the group of infant food, food for elderly, sports nutrition, food for people with special needs, nutritional supplements, dressings, sauces, mayonnaise, low-fat spreads containing less than 30% of fat, creamer.

Infant food is made specifically for infants, babies, roughly between the age of from birth up to two years. It is meant to be a soft and easily consumed food. Particular in these aspects it is similar to food for elderly. Food for elderly is not so much identified by a specific age, rather by the fact that people may prefer softer and easily consumed food.
The food for people with special needs may include foods for diabetics, for people suffering from heart disease, artery disease, kidney disease, and/or liver disease, or people impaired by any other physical diseases or foods for swallowing impaired people and the like.
The food product includes the oleosomes of the present invention in an amount and a form such that the oxidative stability has a positive impact on the chemical and/or organoleptic shelf life time of the product, which is more oxidative stable and/or is less impacted by oxidation catalysts or oxidation precursors. In addition, these products may encounter additional surprising properties such as improved, enhanced or facilitated digestibility.

The dressings, sauces, mayonnaise, low fat spread containing less than 41% of fat, creamer of the present invention are containing the high oleic seed oleosomes of the present invention in an amount of from 5 to up to 80%. The dressings, sauces, mayonnaise, low fat spread containing less than 41% of fat, creamer of the present invention have a good texture, good oxidative stability, and good shelf life time in respect of organoleptic and/or chemical properties.
These types of existing commercial products either contain anti-oxidants in the form of EDTA (disodium ethylenediaminetetra-acetate) or in alternative commercial products EDTA is substituted by herbs and/or extracts with anti-oxidative properties. Typical examples are curcuma and/or rosemary extract. The products with the herb extracts may have an additional expressed unwanted colour and/or taste, while the oxidation induction period is still lower than the oxidation induction period of the commercial product comprising EDTA.
The addition of these anti-oxidants or chelators such as EDTA (ethylenediaminetetra-acetate), which is commonly used in dressing, sauces and mayonnaise, can be reduced, preferably omitted by applying the oleosomes of the present invention. In fact, the dressings, sauces, mayonnaise of the present invention contain a significant reduced amount of EDTA, less than 50 ppm of EDTA, preferably no EDTA.
For all the cases where colour and/or the expressed taste is not a negative aspect, the food products of the current invention, in particular the dressings, sauces, mayonnaise, low fat spread containing less than 41% of fat of the present invention contain the oleosomes of the present invention and may further contain herbs and/or extracts.

The present invention relates to food products wherein the oxidative stability of the food product is obtained by the oleosomes, more in particularly is exclusively obtained by the oleosomes. In fact, the food product of the present invention is a dressing, fat spread containing less than 41% of fat, a mayonnaise, wherein the food product is without further antioxidants. The oleosomes are responsible for the antioxidant properties and no further antioxidants or chelators such as EDTA are added. Herbs or extracts may be present for organoleptic purposes.
The present invention shows that these products comprising the oleosomes have an oxidation induction period which is significantly longer than the oxidation induction period of the commercial products comprising the herbs and/or extracts. The products of the present invention have an oxidation induction period which is as long as or even longer than the oxidation induction period of commercial products comprising EDTA.

The infant food, food for elderly, sports nutrition, food for people with special needs, nutritional supplements of the present invention are containing the high oleic seed oleosomes of the present invention in an amount of from 15 to up to 30%, preferably from 20 to 28%.

The present invention further relates to products that further comprise another oleosomes from a different source than the high oleic seed and/or another liquid oil. In order to match the oxidative induction period of commercial products comprising EDTA it may not be needed to fully substitute the seed oil with the corresponding high oleic seed oleosomes in a 1 to 1 weight ratio. The same level of oxidative stability may be achieved by selecting a suitable blend of high oleic seed oleosomes and another oleosomes from a different source than the high oleic seed and/or another liquid oil with at least 10 %, preferably at least 15%, at least 20%, at least 25%, at least 30%, most preferably at least 40% high oleic oleosomes in the blend, and up to 80%, preferably up to 70%, more preferably up to 60% of high oleic oleosomes in the blend.

Furthermore, the present invention relates to the use of the current oleosomes for their antioxidants properties in food, feed or personal care product.
The present invention relates to the use of the oleosomes for its oxidative stability in food, feed or personal care product. It further relates to the use of the present invention wherein the oxidative stability of the food, feed or personal care product is increased.

More particularly it relates to the use wherein the food product is selected from infant formula, food for elderly, sports food, food for people with special needs, dressing, sauce, mayonnaise, low-fat spreads containing less than 41% fat, or creamer.

Finally it relates to process for preparing the oleosomes of the present invention and it is comprising the following steps:
a) Separating a suspension of seeds and water in a ratio of 1:0.5 up to 1:10, into a hydrophobic-like phase and a hydrophilic-like phase by applying a gravity-based separation force, and
b) Washing at least two times with water in a ratio of 1:0.5 up to 1:10 of the hydrophobic-like phase to water for obtaining the oleosomes,
c) Optionally repeating the step b),
d) and
wherein the suspension has a pH of from 6.5-8, preferably from 7 - 7.8, more preferably from 7.3 - 7.7, from 7.4 to 7.5.

In one aspect of the invention, the process is including step c), alternatively step b) is repeated more than once.

In one aspect of the invention, the process of the present invention is characterized in that the suspension is prepared by soaking the seeds in water over a time of more than 2h, more than 4h, up to 24h.
Furthermore, surprisingly, the inventors have found that in the process of the present invention, the obtained oleosomes are heated, preferably pasteurized, preferably at a temperature of from 80 to 90°C, for a few seconds. The pasteurized oleosomes have a further improved oxidation stability.

In another aspect of the invention, the process of the present invention is comprising the following steps:
a) Taking a suspension of seeds and water in a ratio of 1:0.5 up to 1:10,
b) Bringing pH to 6.5-8, preferably to 7 - 7.8, more preferably to 7.3 - 7.7, from 7.4 to 7.5,
c) Separating the suspension into a hydrophobic-like phase and a hydrophilic-like phase by applying a gravity-based sedimentation force, preferably a centrifugal force at speed of from 5,000 to 40,000 rpm), preferably up to 12,000 rpm
d) Washing at least two times with water in a ratio of 1:0.5 up to 1:10 of the hydrophobic-like phase to water for obtaining the oleosomes,
e) Optionally repeating the step b),
f) Optionally pasteurising at temperature of from 80 to 90°C, up to 20 seconds.

In another aspect of the invention, the process of the present invention is comprising the separating step that is using a centrifuge.

### Analytical methods

Protein content is determined by the ISO 16634 method for measuring crude protein.

Oxidative stability measurement, applying ASTM D525-05 at 110°C and at 5 bar oxygen pressure.

ML (Mikrolab Aarhus) Oxipres™ is an accelerated oxidation test device based on the oxygen consumption of a sample during a certain time and temperature settings.

The (Mikrolab Aarhus) Oxipres™ device was used according to the following the procedure, which is also described in ASTM D525-05:
1.) 20 min before the measurement the heater was set to 110°C (alternatively to 70°C).
2.) The glass vessels of the device were filled with 4 g (in fat content) of sample. The glass cover was placed on top of the vessel.
3.) The covered glass vessel were placed in the pressure vessel. After closing the pressure vessel by hand is was connected to the oxygen filling station.
4.) The outlet pressure at the regulator was adjusted to 5 bar and the pressure vessel was flushed 3 times with Oxygen to bring nitrogen content below 1 %.
5.) The pressure vessel was filled with Oxygen at 4-5 bar. When the pressure was stable, the filling tubes were disconnected and the pressure vessels were placed in the block heater and the test was started in the software.
6.) The pressure inside the pressure vessel is measured and the data is read by the ML Paralog Software and the onset of oxidation or induction period is deducted from drop in oxygen pressure. The time up to the onset of oxidation is the time (in hours) of oxidation stability of the sample

The present invention is illustrated by the following examples.

### Examples

### Example 1 - Preparation of pH adjusted high oleic sunflower seed oleosomes in a 4 step centrifugation:

High oleic sunflower seeds were soaked or preconditioned overnight in demineralized water in ratio 1:3 (solids: water) at 4°C. The soaked seeds were blended in a Vorwerk Thermomix TM5 at the highest speed setting (10,700 rpm) for 1.5 minutes with deionized water in ratio 1:10 (solids: water) and the resulting slurry was filtered through two layers of cheese cloth.
The pH-Value was adjusted to 7.5 with 1 M Sodium hydroxide and the resulting oleosomes was centrifuged for 30 minutes at 5000 rpm, 4°C in a Thermo Scientific Sorvall Legend XFR. After centrifugation, the floating cream layer (oleosomes, oil bodies) was collected with a small metal spoon. For the first washing step, the obtained cream was diluted in *demi water* and centrifuged for 30 minutes at 5000 rpm at 4°C. The obtained cream was diluted in *sodium phosphate buffer* 10 mM at pH 7.5 and centrifuged again for 30 minutes at 5000 rpm at 4°C. Afterwards the obtained cream was dissolved in a *sodium chloride solution* of 1 M and centrifuged again for 30 minutes at 5000 rpm at 4°C. In the last washing step the obtained cream was diluted again in *sodium phosphate buffer* 10 mM at pH 7.5 and centrifuged for 30 minutes at 5000 rpm at 4°C.

Before the Oxipres™ measurement the pH of the obtained cream fraction was adjusted to 3.5 with hydrochloric acid (1 N) and the sample was centrifuged to decrease the amount of water.

The results are shown in Table 1.

**Table 1:**

| | **Induction Period (IP) in hours - measured with ASTM D525-05 in Oxipres at 110°C** |
|---|---|
| Example 1 | 10.5 h |
| Commercial available mayonnaise | 5.3 h |

## Claims

1. Extracted or isolated, oxidative stable high oleic seed oleosomes **characterized in that** the oleosomes are comprising high oleic seed oil with an fatty acid profile comprising at least 65% oleic acid, and the oxidative stability of the oleosomes is increased with at least 50%, preferably at least 70%, more preferably at least 100% to the oxidative stability of the corresponding seed oil.

2. The oleosomes according to claim 1 wherein the seed is sunflower, rapeseed, canola, camelina, olive, safflower, soybean, corn germ, safflower, preferably sunflower.

3. The oleosomes according to claim 1 or 2 wherein the oxidative stability expressed as the oxidation induction period measure with ASTM D525-05 at 110°C and oxygen pressure of 5 bar is at least 8 h, preferably at least 9 h, more preferably at least 10 h .

4. A food, feed or personal care product comprising ingredients and the oleosomes according to anyone of claims 1 to 3.

5. The food, feed or personal care product according to claim 4 **characterized in that** it is containing an analytical detectable amount of oleosin.

6. The food product according to claim 4 or 5 wherein the food product is selected from infant food, food for elderly, sports food, food for people with special needs, dressings, mayonnaise, low-fat spreads containing less than 41% fat, creamer.

7. The food product according to anyone of claims 4 to 6 wherein the oxidative stability of the food product is obtained by the oleosomes.

8. The food product according to anyone of claims 4 to 7 wherein the food product is a dressing, low-fat spread containing less than 41% of fat or a mayonnaise, and wherein the food product is without EDTA.

9. The food product according to anyone of claims 4 to 8 wherein the food product is further comprising another oleosomes from a different source than the high oleic seed and/or another liquid oil.

10. The food product according to claim 9 wherein the high oleic oleosomes are present in the blend in an amount of at least 10 %, preferably at least 15%, at least 20%, at least 25%, at least 30%, most preferably at least 40%, and up to 80%, preferably up to 70%, more preferably up to 60% of high oleic oleosomes in the blend.

11. Use of oleosomes according to claims 1 to 3 for increasing the oxidative stability of the food, feed or personal care product comprising the oleosomes.

12. A process for preparing the oleosomes according to anyone of claims 1 to 3, comprising the following steps:
a) Separating a suspension of seeds and water in a ratio of 1:0.5 up to 1:10, into a hydrophobic-like phase and a hydrophilic-like phase by applying a gravity-based separation force, and
b) Washing at least two times with water in a ratio of 1:0.5 up to 1:10 hydrophobic-like phase to water for obtaining the oleosomes,
c) Optionally repeating the step b),
and
wherein the suspension has a pH of from 6.5-8, preferably from 7 - 7.8, more preferably from7.3 - 7.7, from 7.4 to 7.5.

13. The process according to anyone of claims 12 wherein the suspension is prepared by soaking the seeds in water over a time of more than 2h, more than 4h, up to 24h.

14. The process according to anyone of claims 12 to 13 and the process is comprising the following steps
a) Taking a suspension of seeds and water in a ratio of 1:0.5 up to 1:10,
b) Bringing pH to 6.5-8, preferably to 7 - 7.8, more preferably to 7.3 - 7.7,
c) Separating the suspension into a hydrophobic-like phase and a hydrophilic-like phase by applying a sedimentation force, preferably a centrifugal force at high speed (5,000 - 40,000 rpm), preferably up to 12,000 rpm,
d) Washing at least two times with water in a ratio of 1:0.5 up to 1:10 of the hydrophobic-like phase to water for obtaining the oleosomes,
e) Optionally pasteurising at temperature of from 80 to 90°C, up to 20 seconds.

15. The process according to anyone of claims 12 to 14 wherein the separating step is using a centrifuge.
